# EUROPÄISCHE PATENTSCHRIFT

(11) **EP 3 199 144 B1**
(45) Veröffentlichungstag und Bekanntmachung des Hinweises auf die Patenterteilung: **27.02.2019**
(21) Anmeldenummer: 17153191.6
(22) Anmeldetag: 26.01.2017
(51) Int. Cl.: A61K 9/00, A61K 47/14, A61K 9/06, A61K 47/36, A61K 9/50, A61K 9/51, A61K 31/00, A61K 33/40

(54) **PHARMAZEUTISCHE ZUSAMMENSETZUNG ZUR BEHANDLUNG PARODONTALER ODER PERIIMPLANTÄRER ERKRANKUNGEN**
PHARMACEUTICAL COMPOSITION FOR THE TREATMENT OF PARODONTAL OR PERI-IMPLANTAL DISEASES
COMPOSITION PHARMACEUTIQUE POUR LE TRAITEMENT DE MALADIES PARODONTIQUES OU PERI-IMPLANTAIRES

(30) Priorität: 26.01.2016 DE 102016101280
(43) Veröffentlichungstag der Anmeldung: 02.08.2017
(73) Patentinhaber: Bröseler, Frank, 52070 Aachen (DE)
(72) Erfinder: Bröseler, Frank, 52070 Aachen (DE)
(74) Vertreter: Bauer, Dirk

(56) Entgegenhaltungen:
- EP-A1- 0 244 118
- DATABASE MEDLINE [Online] US NATIONAL LIBRARY OF MEDICINE (NLM), BETHESDA, MD, US; 2011, DUNLAP TANYA ET AL: "Subgingival delivery of oral debriding agents: a proof of concept.", XP002770326, Database accession no. NLM22403980
- VYAS S P ET AL: "CONTROLLED AND TARGETED DRUG DELIVERY STRATEGIES TOWARDS INTRAPERIODONTAL POCKET DISEASES", JOURNAL OF CLINICAL PHARMACY AND THERAPEUTICS, BLACKWELL SCIENTIFIC PUBLICATION, OXFORD, GB, Bd. 25, Nr. 1, 1. Februar 2000 (2000-02-01), Seiten 21-42, XP000982055, ISSN: 0269-4727, DOI: 10.1046/J.1365-2710.2000.00261.X
- TARUN GARG ET AL: "Scaffold: A Novel Carrier for Cell and Drug Delivery", CRITICAL REVIEWS IN THERAPEUTIC DRUG CARRIER SYSTEMS, Bd. 29, Nr. 1, 1. Januar 2012 (2012-01-01) , Seiten 1-63, XP55135597, US ISSN: 0743-4863, DOI: 10.1615/CritRevTherDrugCarrierSyst.v29.i1. 10
- Franke ET AL: "Periimplantitis - eine neue Herausforderung", , 2 January 2013 (2013-01-02), pages 69-75, XP055425438, Internet: Research Gate Retrieved from the Internet: URL:file:///C:/Users/JK52031/Desktop/Frank e_2013_paro_2013_01_s0069.pdf [retrieved on 2017-11-15]

## Beschreibung

Die Erfindung betrifft eine pharmazeutische Zusammensetzung zur topischen Anwendung bei der Behandlung parodontaler und/oder periimplantärer Erkrankungen bei Menschen oder Säugetieren.

Bei Parodontitis handelt es sich um eine entzündlich-degenerative Erkrankung des Parodontiums (Zahnhalteapparat, Attachment). Parodontitis ist dabei nicht nur durch eine bakterielle Infektion gekennzeichnet, sondern als Begleiterscheinungen treten die Ausprägung unphysiologisch tiefer "Taschen" (Räume zwischen den Zähnen und dem umgebenden Zahnfleisch) sowie ein alveolärer Knochenabbau auf, der letztlich zu einem Zahnverlust führen kann. Periimplantitis manifestiert sich in einem Verlust der hartgewebigen (knöchernen) Abstützung um ein Zahnimplantat, welches als künstliche Zahnwurzel funktioniert, kombiniert mit einer entzündlichen Reaktion der periimplantären Weichgewebe (med.: Mukositis). Dabei sind im Wesentlichen ähnlich strukturierte mikrobielle Biofilme beteiligt wie die bei der Parodontitis.

Auf festen Oberflächen innerhalb einer umgebenden Flüssigkeit angesiedelte Bakterien aggregieren sich zu einem so genannten Biofilm, der in einer Polysaccharidmatrix mit anderen organischen und anorganischen Materialien gebunden ist. Derartige Komplexe an Zahnoberflächen oder auch Implantatoberflächen werden oraler Biofilm oder Plaque genannt.

Das Hauptziel bei der Behandlung von Parodontitis und Periimplantitis ist die Reduktion der bakteriellen Belastung im Parodontium. Da die Bakterien in besagtem Biofilm angeordnet sind, ist eine gründliche Mundhygiene sowie ein mechanisches Entfernen der Plaque ein Hauptansatz bei der Behandlung parodontaler Behandlungen. Darüber hinaus ist aber auch die lokale medikamentöse Behandlung Stand der Technik: So ist in der WO 92/00718 A ein System zur kontrollierten Freisetzung chemotherapeutischer Stoffe an bestimmten Stellen im Mundraum beschrieben, und zwar zur Behandlung parodontaler Krankheiten. Die vorbekannte Methode besteht in der Platzierung eines Polymer-Liefersystems direkt im Zahnfleischgewebe. Ausdrücklich soll nach der Lehre dieser Anmeldung die Einbringung der pharmazeutisch wirksamen Stoffe nicht in der Alveolartasche, d.h. dem Raum zwischen dem infizierten alveolären Gewebe und dem Zahn, erfolgen. Das vorgenannte Liefersystem kann z.B. Mikrokapseln aufweisen, aber auch Mikrokugeln, Liposomenfasern, Stäbchen, Filme und Ähnliches, in denen jeweils der pharmazeutisch wirksame Stoff untergebracht ist. Als Kapselmaterial ist entweder biologisch abbaubares oder biologisch nicht abbaubares Polymer erwähnt, wobei erstgenanntes bevorzugt wird, da eine Entfernung nach dem Behandlungsende entfällt. Der freizusetzende Wirkstoff soll entweder antimikrobielle Eigenschaften besitzen oder eine entzündungshemmende Wirkung entfalten. Über einen Fluss von Serum aus dem Zahnfleischgewebe in die Alveolartasche sollen hinreichende Wirkstoffkonzentrationen zur Abtötung der Bakterien sowohl im Gewebe als auch in der Tasche geschaffen werden. In diesem Zusammenhang werden insbesondere Vorteile gegenüber Liefersystemen beschrieben, die in der Alveolartasche selbst angeordnet sind. Aber auch sei die Applikation im Zahnfleischgewebe selbst deshalb vorteilhaft, weil die normalen Zahnhygienevorgänge dann eine unbeabsichtigte Entfernung der Wirkstoffe vom Behandlungsort nicht bewirken können.

Darüber hinaus offenbart die WO 96/13253 A den Einsatz eines Antibiotikums des Tetracyclin-Typs zur Behandlung parodontaler Erkrankungen. Es wird ein biologisch abbaubares Liefersystem zur Freisetzung der Wirkstoffe beschrieben. Dabei besteht ein Kern aus einer Natrium-Alginat-Einheit und dem pharmazeutischen Wirkstoff. Die Oberfläche soll mit Chitosan beschichtet sein. Auch die Verwendung von Mikrokapseln mit Polysaccharid ist erwähnt. Als Wirkstoffe sind entweder diverse Antibiotika aufgeführt, aber alternativ auch Ibuprofen und Fluriprofen.

Ferner ist aus der WO 2013/166459 A eine orale Zusammensetzung mit einem biokompatiblen Chelatbildner in einer Mindestkonzentration von 0,1 Gew.-% bekannt. Darüber hinaus enthält die bekannte Zusammensetzung einen Stoff, der die Durchdringung verbessern soll. Die bekannte Zusammensetzung soll mit einem Liefersystem in Form einer liposomalen Dispersion kombiniert werden. Auch sind kolloidale Suspensionen von Mikrokapseln und Nanokapseln genannt. Aufgrund der Biofilmeigenschaften der Plaque wird eine Bekämpfung der die Parodontitis oder Periimplantitis verursachenden Bakterien mit Hilfe antibiotischer Wirkstoffe abgelehnt. Vielmehr soll eine Kreuzreaktivität zwischen unterschiedlichen funktionalen Bestandteilen der Plaque bekämpft und zerstört werden, wozu die vorgenannten Komplexbildner, insbesondere EDTA, verwendet werden sollen.

Aus der US 8449918 B2 ist eine pharmazeutische Zusammensetzung zur topischen Anwendung unter anderem bei der Behandlung von Zahnkrankheiten bekannt. Die vorbekannte Zusammensetzung enthält einen Trägerstoff, in den eine Vielzahl von Mikrokapseln eingebettet sind, wobei die Mikrokapseln jeweils eine Kern-Mantel-Struktur aufweisen. Im Inneren der Mikrokapseln befindet sich mindestens ein Wirkstoff, der durch die Unterbringung in den Mikrokapseln vor der topischen Anwendung geschützt und nach Beginn der Anwendung kontrolliert und über einen längeren Zeitraum am Behandlungsort freigesetzt werden soll. Der Hauptanwendungsfall der vorbekannten Zusammensetzung ist die Behandlung von Akne und zu diesem Zweck wird als bevorzugter, in die Mikrokapseln einzubringender Wirkstoff Benzoyl-Peroxid vorgeschlagen, das ohne die Einkapselung einerseits eine unzureichende Stabilität besäße und andererseits weitere in der Zusammensetzung befindliche Wirkstoffe aufgrund seiner großen Reaktivität zerstören bzw. zumindest schädigen könnte. Neben der Behandlung von Akne ist die Verwendung der vorbekannten Mikrokapseln mit darin enthaltenem Benzoyl-Peroxid auch als antibakterielles und bleichendes Mittel in Zahnpasta vorgeschlagen.

Nach der Lehre der US 8449918 B2 kann auch in dem Trägerstoff ein weiterer, d.h. nicht verkapselter, Wirkstoff enthalten sein. Dabei sollen der nicht verkapselte Wirkstoff und der in den Mikrokapseln enthaltene Wirkstoff miteinander chemisch reaktiv sein. In diesem Zusammenhang sind eine Vielzahl von möglichen Wirkstoffen benannt, die in den Mikrokapseln oder in unverkapselter Form in dem Trägerstoff vorhanden sein können. Antiseptisch wirkende Stoffe sind in der US 8449918 nicht als solche benannt. Sofern Benzoyl-Peroxid als antiseptisch wirkender Stoff anzusehen ist, ist dessen Verwendung ausschließlich in verkapselter Form vorgesehen. Hierin ist gerade das Wesen der Lehre der US 8449918 B2 zu sehen, um Interaktionen des Benzoyl-Peroxid mit eventuell weiteren, in dem Trägerstoff enthaltenen, Wirkstoffen vor der Anwendung sicher zu verhindern.

Darüber hinaus sind aus den Druckschriften WO 90/00048 A1, US 5500228 und US2013/0195953 A1 jeweils Liefersysteme für Wirkstoffe bekannt, bei denen für den Fall einer topischen Anwendung eine zeitlich gesteuerte Freisetzung des Wirkstoffs aus dem Liefersystem erreicht werden soll. Gemäß der WO 90/00048 A1 soll das Liefersystem in einen viskosen Trägerstoff in Form eines Thermogels eingebettet sein. Das Liefersystem mit zeitlich verzögerter Freisetzung besteht aus Mikrokapseln in Form eines semipermeablen anisotopischen Salzfilms, der durch eine Reaktion einer Lewis-Säure oder eines Salzes davon mit einer Lewis-Lauge oder einem Salz davon, gebildet ist. Innerhalb des Trägerstoffs kann ein weiterer Wirkstoff enthalten sein, der sich von dem mikroverkapselten Wirkstoff unterscheiden oder mit diesem identisch sein kann. Als Wirkstoffe sind insbesondere Antibiotika benannt.

Ferner sind in der US 5500228 noch "trockene" Mikrokapseln offenbart, die nicht in einen gesonderten Trägerstoff eingebettet sind. Bei den mikroverkapselten Wirkstoffen kann es sich entweder um ein Antibiotikum oder ein Antiseptikum oder andere Wirkstoffe handeln. Das die Hülle der Mikrokapseln bildende Polymer soll durch Kontakt mit Feuchtigkeit eine klebrige Eigenschaft erhalten und an dem Gewebe, mit dem es in Kontakt kommt, anhaften und auf diese Weise vor zu schneller Auswaschung geschützt sein. Wiederum haben die Mikrokapseln die Aufgabe, einerseits den darin enthaltenen Wirkstoff vor äußeren Einflüssen bis zum Zeitpunkt der Anwendung zu schützen und zum anderen eine kontrollierte Freisetzungsrate über die Zeit zu gewährleisten. Da kein Trägerstoff um die Mikrokapseln herum vorhanden ist, kann auch kein unverkapselter Wirkstoff in der vorbekannten pharmazeutischen Zusammensetzung enthalten sein. Das Dokument EP 0244118 offenbart parodontale Zubereitungen mit kontrollierter Wirkstofffreigabe beinhaltend a) verkapselte Wirkstoffe als Mikropartikel und b) ein Suspendiermedium für die Mikropartikel. Schließlich offenbart die US 2013/0195953 A1 noch ein Verfahren zur Behandlung von Entzündungen, bei dem in Mikrokapseln in einem Trägerstoff in Form eines Gels suspendiert sein können. Bei dem vorbekannten Verfahren soll die topische Anwendung der pharmazeutischen Zusammensetzung auch zur Behandlung einer Entzündung in Folge von Parodontose erfolgen. Insbesondere sollen als Wirkstoffe in der bekannten Zusammensetzung ein NSAID oder Resolvin zur Anwendung kommen. Die Verwendung von Antibiotika oder Antiseptika ist in der US 2013/0195953 A1 nicht vorgeschlagen.
Sämtliche vorbekannten Behandlungsmethoden und die dazu verwendeten Wirkstoffe haben sich leider als nur bedingt wirksam herausgestellt.

### Aufgabe

Der Erfindung liegt die Aufgabe zugrunde, eine pharmazeutische Zusammensetzung bereitzustellen, mit der der Behandlungserfolg gegenüber dem Stand der Technik verbessert werden kann.

### Lösung

Die erfindungsgemäße Lösung der vorgenannten Aufgabe besteht in einer pharmazeutischen Zusammensetzung zur topischen Anwendung bei der Behandlung parodontaler und/oder periimplantärer Erkrankungen bei Menschen oder Säugetieren, umfassend
- mindestens einen antiseptisch wirkenden Stoff,
- mindestens einen antibiotisch wirkenden Stoff, der sich in Kapseln befindet, die nach der Applizierung den enthaltenen antibiotisch wirkenden Stoff zeitlich gesteuert freisetzen,
- mindestens einen Trägerstoff, in dem sich der mindestens eine antiseptisch wirkende Stoff und die den mindestens einen antibiotisch wirkenden Stoff enthaltenden Kapseln befinden,
wobei die Zusammensetzung bei einer Raumtemperatur von 20°C eine dynamische Viskosität zwischen 5 mPas und 10⁵ mPas, vorzugsweise zwischen 50 mPas und 10⁴ mPas, weiter vorzugsweise zwischen 100 mPas und 10⁴ mPas aufweist.
Ein wesentliches Merkmal der Erfindung wird von der Kombination eines antiseptisch wirkenden Stoffs mit einem antibiotisch wirkenden Stoff gebildet. Dabei hat der antiseptisch wirkende Stoff die Aufgabe, zu Beginn der Behandlung insbesondere nach einer vorangegangenen oder gleichzeitigen mechanischen Entfernung des Biofilms eine Desinfektion der zu behandelnden Stelle oder Bereiche zu bewirken und den Biofilm und die darin vorhandenen Bakterien am Behandlungsort zu inaktivieren oder zu zerstören. Diese Maßnahme stellt den ersten Schritt bei der Anwendung der erfindungsgemäßen Zusammensetzung dar. Deshalb darf sich der antiseptisch wirkende Stoff auch nicht ausschließlich in den Kapseln befinden, bzw. wenn sich ein Teil davon zusätzlich zu dem antibiotisch wirkenden Stoff darin befindet, muss ein weiterer Teil sich in dem Träger befinden, damit er an der zu behandelnden Stelle unmittelbar zu Behandlungsbeginn zur Verfügung steht. Die zweite wesentliche Komponente ist der antibiotisch wirkende Stoff, der auf die nach der Desinfektion verbliebenen Mikroorganismen einwirkt und deren Neuentwicklung unterbindet bzw. minimiert (med.: Bakterizidie, Bakteriostase). Im Hinblick auf den antibiotisch wirkenden Stoff ist insbesondere die zeitliche Komponente der Zurverfügungstellung entscheidend. So ist es nicht ausreichend, den antibiotisch wirkenden Stoff so zu applizieren, dass er einmalig vom Applikationszeitpunkt an sofort bzw. sehr kurzfristig in der gesamten Menge am Behandlungsort zur Verfügung steht. Vielmehr ist eine zeitlich verzögerte Freisetzung wichtig, die dafür sorgt, dass über einen längeren Zeitraum der Behandlung, typischerweise eine Mehrzahl von Tagen oder Wochen, eine möglichst gleichmäßige Freisetzung des mindestens einen antibiotisch wirkenden Stoffs gewährleistet ist. Dabei kommt es nicht auf eine exakt konstante Freisetzungsrate über einen bestimmten Zeitraum an, sondern eine intermittierende Freisetzung bestimmter (jeweils gleicher oder unterschiedlicher) Wirkstoffmengen ist gleichfalls denkbar und einem Behandlungserfolg nicht abträglich. Sinnvollerweise wird mit der Freisetzung des mindestens einen antibiotisch wirkenden Stoffs unmittelbar nach der Applikation der erfindungsgemäßen Zusammensetzung begonnen, es ist aber auch möglich, zunächst eine gewisse Initialphase ohne antibiotische Behandlung vorzusehen, um zunächst die Desinfektionsphase mit Hilfe des mindestens einen antiseptisch wirkenden Stoffs abzuschließen bzw. deren Erfolg eintreten zu lassen. So können Wechselwirkungen zwischen dem Antiseptikum und dem Antibiotikum bei Bedarf vermieden werden.

Ein weiterer wesentlicher Aspekt der Erfindung besteht in der Realisierung einer hinreichend großen Viskosität, damit die applizierte Zusammensetzung möglichst lange am Behandlungsort, d.h. insbesondere innerhalb einer Zahnfleischtasche, verbleibt und nicht durch Speichel, körpereigenes Sekret, Nahrung oder andere Einflüsse zu frühzeitig ausgetragen wird. Die erfindungsgemäß vorgeschlagene Konsistenz bei der Applizierung entspricht in etwa der von Honig oder Sirup, erlaubt also eine gewisse Verteilung und ein "zähes Fließen" aus einem Applikationsinstrument und Verteilen innerhalb der Zahntasche, unterbindet allerdings ein ungewolltes und rasches Austreten aus derselben.

Erfindungsgemäß kann vorgesehen sein, dass sich die Viskosität der Zusammensetzung nach der Applizierung in Folge einer Wechselwirkung mit Körperflüssigkeit und/oder durch die Erhöhung von Raumtemperatur auf physiologische Körpertemperatur (ca. 36°C bis 38°C) weiter erhöht. Nach der Verteilung in der parodontalen oder periimplantären Tasche ist eine solche Viskositätssteigerung hilfreich, da die Gefahr eines unerwünschten Austritts oder Auswaschens weiter reduziert wird. Die Viskosität nach Abschluss der durch die Körperflüssigkeit und/oder Temperatursteigerung induzierten Verhärtung könnte im Bereich von etwa 10³ bis 10⁶ mPas liegen, was einer "pastösen" Konsistenz entspricht. Die Realisierung einer Viskosität in dem vorgenannten Intervall vor der Applikation ist ungünstig, da eine gleichmäßige Verteilung in der Zahntasche dann nicht sichergestellt werden kann bzw. die Applizierung mit gebräuchlichen Instrumenten erschwert wird.

Die nachträgliche Viskositätssteigerung kann dabei darauf basieren, dass sich durch Erhöhung der Temperatur (von Raum- bzw. Kühltemperatur bei Lagerung auf Körpertemperatur) Ausfällungen bilden, welche den Trägerstoff innerhalb der Zusammensetzung in seiner Fließfähigkeit verändern.

Im Zusammenhang mit der nachträglichen Viskositätssteigerung der Zusammensetzung ist es allerdings von Bedeutung, dass sowohl der Vorgang der zeitlich gesteuerten Freisetzung des antibiotisch wirkenden Stoffs als auch die Diffusion dieses Wirkstoffs aus der Zusammensetzung in das umgebende Gewebe möglichst nicht behindert, jedenfalls nicht gänzlich unterbunden wird.

Gemäß einer Ausgestaltung der Erfindung kann der mindestens eine antiseptische Stoff einer der folgenden Stoffklassen zugehörig sein:
- Alkohole
- Peroxide
- Jod- oder chlorhaltige Verbindungen
- Chinolin-Derivate
- Benzochinon-Derivate

Gemäß einer Ausgestaltung der erfindungsgemäßen Zusammensetzung können die Kapseln Nano- und/oder Mikrokapseln sein. Ein möglicher Stoff für die Kapseln ist z.B. ein Polysaccharid. Gegebenenfalls kann eine äußere Beschichtung auf die Kapseln aufgebracht sein.

Es ist als vorteilhaft anzusehen, wenn bei einem ersten Teil der Kapseln eine Freisetzung des enthaltenen, mindestens einen antibiotisch wirkenden Stoffs in einem Zeitraum zwischen 1 Tag und 10 Tagen ab der Applikation erfolgt, wohingegen bei einem möglichen zweiten Anteil der Kapseln eine Freisetzung in einem Zeitraum zwischen 1 Tag und 20 Tagen ab der Applikation erfolgt. Es können auch mehr als zwei Anteile mit einer unterschiedlichen Freisetzungscharakteristik verwendet werden. Dies kann insbesondere bei sehr langen Behandlungsdauern sinnvoll sein. Es kann sinnvoll sein, in die Kapseln mit den unterschiedlichen Freisetzungscharakteristika denselben antibiotisch wirkenden Stoff einzubringen, oder aber verschiedene antibiotisch wirkende Stoffe. Auch eine Kombination ist denkbar, so z.B. in dem Sinne, dass ein erster antibiotisch wirkender Stoff in zwei oder mehr Kapselarten mit unterschiedlichen Freisetzungscharakteristika enthalten ist und ein zweiter oder auch dritter antibiotisch wirkender Stoff ebenfalls jeweils in zwei oder mehr Kapselarten mit unterschiedlichen Freisetzungscharakteristika untergebracht ist.

Eine Weiterbildung der Erfindung besteht darin, dass der Trägerstoff ein Gel, Alginat, eine Lipidzusammensetzung oder eine Mischung aus den vorgenannten Stoffen ist. Als Zusatzstoffe zum Trägerstoff können Naturharze eingesetzt werden, um die Konsistenz zu beeinflussen, insbesondere die Viskosität zu erhöhen. Bei der Fettzusammensetzung kann es sich auch um eine Emulsion handeln, insbesondere eine solche mit durchgehender Wasserphase, um die Durchgängigkeit für den mindestens einen antibiotisch wirkenden Stoff und/oder dem mindestens einen antiseptisch wirkenden Stoff sicher zu stellen.

Offenbart ist weiterhin ein Verfahren zur Behandlung parodontaler und/oder periimplantärer Erkrankungen bei Menschen oder Säugetieren unter Verwendung einer pharmazeutischen Zusammensetzung mit
- mindestens einen antiseptisch wirkenden Stoff,
- mindestens einen antibiotisch wirkenden Stoff, der sich in Kapseln befindet, die nach der Applizierung den enthaltenen antibiotisch wirkenden Stoff zeitlich gesteuert freisetzen, mindestens einen Trägerstoff, in dem sich der mindestens eine antiseptisch wirkende Stoff und die den mindestens einen antibiotisch wirkenden Stoff enthaltenden Kapseln befinden,
wobei die Zusammensetzung bei Raumtemperatur eine dynamische Viskosität zwischen 5 mPas und 10⁵ mPas aufweist, gekennzeichnet durch die folgenden Verfahrensschritte:
a) Im Bereich einer parodontalen oder periimplantären Tasche wird ein Biofilm mit zumindest teilweise pathogenen Mikroorganismen zerstört und/oder entfernt
b) In die parodontale oder periimplantäre Tasche wird die vorgenannte pharmazeutische Zusammensetzung eingebracht, so dass sie zumindest mit den gemäß a) gereinigten Bereichen der Oberfläche der Zahnwurzel oder des Implantats in Kontakt kommt.
Die Zerstörung und/oder Entfernung des Biofilms gemäß a) kann mit mindestens einem mechanischen und/oder chemischen Mittel erfolgen.

### Ausführungsbeispiel

Die Erfindung wird nachfolgend anhand eines Ausführungsbeispiels einer pharmazeutischen Zusammensetzung und deren Applikation in eine Zahntasche, die in der Zeichnungsfigur schematisch dargestellt ist, näher erläutert.

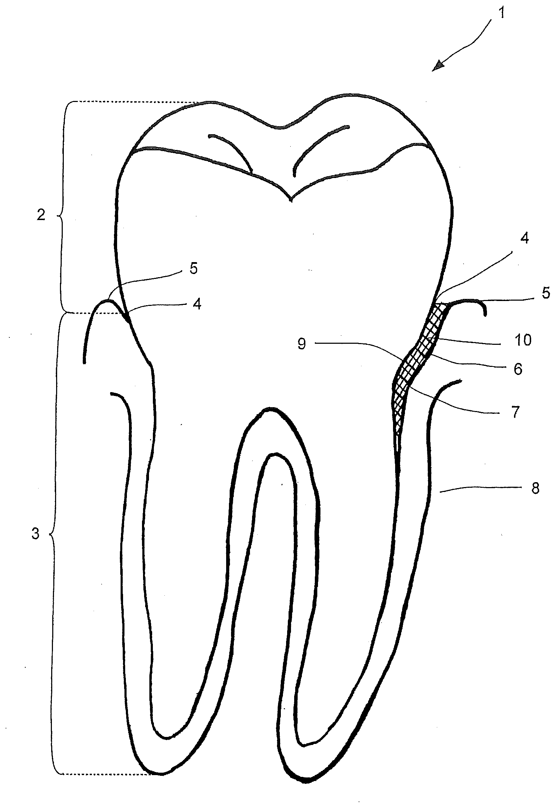

Die Zeichnung zeigt einen schematischen Schnitt durch einen menschlichen Kiefer. Ein Zahn 1 lässt sich unterteilen in eine Zahnkrone 2 und eine Zahnwurzel 3, wobei eine den Zahn 1 in Umfangsrichtung umschließende Grenzlinie 4 die Zahnkrone 2 von der Zahnwurzel 3 abgrenzt. Bei der Zahnkrone 2 handelt es sich somit um den - bei gesunden, morphologisch regulären Verhältnissen - im Mundraum klinisch sichtbaren Teil des Zahnes 1. An einer weiteren Grenzlinie 6 geht die Gingiva 5 in das innere Saumepithel 7 über. Die Verbindung zwischen der Zahnwurzel 3 und dem paradontalen Knochen 8 wird durch einen Halteapparat aus Fasern hergestellt.

Die erfindungsgemäße pharmazeutische Zusammensetzung wird mittels eines geeigneten Applikationsinstruments (z.B. Kanüle) in eine zwischen einer Oberfläche 9 der Zahnwurzel 3 einerseits und der Gingiva 5 bzw. des inneren Saumepithels 7 andererseits befindliche parodontale oder periimplantäre Tasche 10 eingebracht. Vor der Einbringung wird ein an der Oberfläche 9 der Zahnwurzel anhaftender Biofilm mechanisch entfernt, und/oder chemisch zerstört um die Bakterienbeladung der Tasche 10 zu reduzieren und möglichst gute Anfangsbedingungen für die anschließende medikamentöse Behandlung zu schaffen.

Die pharmazeutische Zusammensetzung weist als Trägerstoff, wie vorbeschrieben, ein Gel, Alginat, eine Lipidzusammensetzung oder eine Mischung aus den vorgenannten Stoffen auf. Als Zusatzstoffe im Trägerstoff können Naturharze eingesetzt werden, um die Konsistenz zu beeinflussen. Aus der Stoffklasse der Alginate/Alginatenderivate kommen insbesondere Propylenglycolalginate [PGA] in Betracht, da diese synthetisierbar sind und bereits seit langem erfolgreich als Trägersubstanzen zur kontrollierten Medikamentenfreisetzung, zum Beispiel bei sog. Retard-Tabletten, zum Einsatz kommen.

In dem Trägerstoff befinden sich Mikro- oder Nanokapseln als eigenständige Träger für den mindestens einen antibiotisch wirkenden Stoff. Diese aus einem Polysaccharid bestehenden Mikro- oder Nanokapseln haben eine kugel- oder eiförmige 3-dimensionale Gestalt von 100 bis 10.000 nm Durchmesser. Mikro- oder Nanokapseln lassen sich synthetisieren und können dem Trägerstoff, welcher sich gegenüber den Mikro- oder Nanokapseln chemisch neutral zu verhalten hat (keine Lyse der Mikro- oder Nanokapseln durch den Trägerstoff), beigegeben werden. Darüber hinaus befindet sich in dem Trägerstoff ein antiseptisch wirkender Stoff in Form von Wasserstoffperoxid.

Die dynamische Viskosität der Zusammensetzung beträgt vor Applikation bei Raumtemperatur (20°C) ca. 100 mPas. Infolge der Einwirkung von Körpertemperatur und/oder Köperserum auf den Trägerstoff kommt es innerhalb weniger Stunden nach der Applikation zu einer Viskositätssteigerung auf ca. 10⁴ mPas bis 10⁵ mPas

Die Behandlung bei einer Periimplantitis erfolgt in analoger Weise zu der vorstehend erläuterten Vorgehensweise bei Parodontitis, nur dass die Einbringung in eine periimplantäre Tasche zwischen einer nicht mehr osseointegrierten Implantatoberfläche und dem entzündlich veränderten periimplantären Weichgewebe erfolgt.

### Bezugszeichenliste

- 1: Zahn
- 2: Zahnkrone
- 3: Zahnwurzel
- 4: Grenzlinie
- 5: Gingiva
- 6: Grenzlinie
- 7: inneres Saumepithel
- 8: Kieferknochen
- 9: Oberfläche
- 10: Parodontale Tasche

## Patentansprüche

1. Pharmazeutische Zusammensetzung zur topischen Anwendung bei der Behandlung parodontaler oder periimplantärer Erkrankungen bei Menschen oder Säugetieren umfassend
- mindestens einen antiseptisch wirkenden Stoff,
- mindestens einen antibiotisch wirkenden Stoff, der sich in Kapseln befindet, die nach der Applizierung den enthaltenen antibiotisch wirkenden Stoff zeitlich gesteuert freisetzen,
- mindestens einen Trägerstoff, in dem sich zumindest ein Teil des antiseptisch wirkenden Stoffs befindet.
wobei die Zusammensetzung bei einer Raumtemperatur von 20°C eine dynamische Viskosität zwischen 5 und 10⁵ mPas, vorzugsweise zwischen 50 und 10⁴ mPas, weiter vorzugsweise zwischen 100 und 10⁴ mPas aufweist.

2. Pharmazeutische Zusammensetzung nach Anspruch 1, **dadurch gekennzeichnet, dass** der mindestens eine antiseptisch wirkende Stoff
- ein Alkohol und/oder
- eine quartäre Amoniumverbindung und/oder
- eine jodhaltige Verbindung und/oder
- eine halogenierte Verbindung und/oder
- eine quecksilberhaltige Verbindung
ist.

3. Pharmazeutische Zusammensetzung nach Anspruch 1 oder 2, **dadurch gekennzeichnet, dass** der mindestens eine antibiotisch wirkende Stoff mindestens eine der folgenden Wirkungsmechanismen aufweist:
- Hemmung der Zellwandsynthese,
- Hemmung der Proteinbiosynthese am Ribosom,
- Wirkung auf bakterielle Nukleinsäuren

4. Pharmazeutische Zusammensetzung nach einem der Ansprüche 1 bis 3, **dadurch gekennzeichnet, dass** die Kapseln Nano- und/oder Mikrokapseln sind.

5. Pharmazeutische Zusammensetzung nach einem der Ansprüche 1 bis 4, **dadurch gekennzeichnet, dass** die Kapseln aus einem Polysaccharid bestehen.

6. Pharmazeutische Zusammensetzung nach einem der Ansprüche 1 bis 5, **dadurch gekennzeichnet, dass** die Freisetzung des in den Kapseln enthaltenen, mindestens ein antibiotisch wirkenden Stoffs über einen Zeitraum von mindestens 5 Tagen, vorzugsweise mindestens 10 Tagen erfolgt.

7. Pharmazeutische Zusammensetzung nach einem der Ansprüche 1 bis 6, **dadurch gekennzeichnet, dass** bei einem ersten Teil der Kapseln eine Freisetzung des enthaltenen mindestens einen antibiotisch wirkenden Stoffs in einem Zeitraum zwischen einem Tag und 10 Tagen ab der Applikation und/oder bei einem zweiten Anteil der Kapseln eine Freisetzung des enthaltenen mindestens einen antibiotisch wirkenden Stoffs in einem Zeitraum zwischen einem Tag und 20 Tagen und/oder bei einem dritten Anteil der Kapseln eine Freisetzung des enthaltenen mindestens einen antibiotisch wirkenden Stoffs in einem Zeitraum zwischen 10 Tagen und 20 Tagen, erfolgt.

8. Pharmazeutische Zusammensetzung nach einem der Ansprüche 1 bis 7, **dadurch gekennzeichnet, dass** der Trägerstoff vorzugsweise ein Gel oder eine Fettzusammensetzung oder eine Mischung aus den vorgenannten Stoffen ist.

9. Pharmazeutische Zusammensetzung nach einem der Ansprüche 1 bis 8, **dadurch gekennzeichnet, dass** die Viskosität der Zusammensetzung nach der Applizierung durch eine Wechselwirkung mit Körperflüssigkeit und/oder der Körpertemperatur ansteigt, wobei vorzugsweise die Viskosität nach Eintritt der Wechselwirkung mit der Körperflüssigkeit und/oder Körpertemperatur, vorzugsweise nach einem Zeitraum von einem Tag bis drei Tagen, zwischen 10³ mPas und 10⁶ mPas beträgt.

10. Pharmazeutische Zusammensetzung nach Anspruch 9, **dadurch gekennzeichnet, dass** der Anstieg der Viskosität durch eine Versalzung im Anschluss an eine durch Körperflüssigkeit bedingte Hydrolisierung der Zusammensetzung, insbesondere des Trägerstoffs, insbesondere des als Gel ausgebildeten Trägerstoffs, erfolgt.

## Claims

1. A pharmaceutical composition for topical use in the treatment of periodontal or peri-implant diseases in human beings or mammals, comprising
- at least one substance with an antiseptic action,
- at least one substance with an antibiotic action which is disposed in capsules which, after application, provide delayed release of the substance with an antibiotic action contained therein,
- at least one support which contains at least a portion of the substance with an antiseptic action,
wherein the composition has a dynamic viscosity in the range 5 to 10⁵ mPas, preferably in the range 50 to 10⁴ mPas, more preferably in the range 100 to 10⁴ mPas at an ambient temperature of 20°C.

2. The pharmaceutical composition as claimed in claim 1, **characterized in that** the at least one substance with an antiseptic action is:
- and alcohol, and/or
- a quaternary ammonium compound, and/or
- an iodine-containing compound, and/or
- a halogenated compound, and/or
- a mercury-containing compound.

3. The pharmaceutical composition as claimed in claim 1 or claim 2, **characterized in that** the at least one substance with an antibiotic action has at least one of the following modes of action:
- inhibition of cell wall synthesis,
- inhibition of protein biosynthesis at the ribosome,
- action on bacterial nucleic acids.

4. The pharmaceutical composition as claimed in one of claims 1 to 3, **characterized in that** the capsules are nano- and/or micro-capsules.

5. The pharmaceutical composition as claimed in one of claims 1 to 4, **characterized in that** the capsules consist of a polysaccharide.

6. The pharmaceutical composition as claimed in one of claims 1 to 5, **characterized in that** the at least one substance with an antibiotic action contained in the capsules is released over a time period of at least 5 days, preferably at least 10 days.

7. The pharmaceutical composition as claimed in one of claims 1 to 6, **characterized in that** for a first portion of the capsules, the at least one substance with an antibiotic action contained therein is released over a time period in the range from one day to 10 days following application, and/or for a second portion of the capsules, the at least one substance with an antibiotic action contained therein is released over a time period in the range from one day to 20 days, and/or for a third fraction of the capsules, the at least one substance with an antibiotic action contained therein is released over a time period in the range from 10 days to 20 days.

8. The pharmaceutical composition as claimed in one of claims 1 to 7, **characterized in that** the support is preferably a gel or a lipid composition or a mixture of said substances.

9. The pharmaceutical composition as claimed in one of claims 1 to 8, **characterized in that** following application, the viscosity of the composition increases by means of an interaction with body fluid and/or the body temperature, wherein, after a period of time following onset of the interaction with the body fluid and/or the body temperature, preferably after a period of time in the range from one day to three days, the viscosity is preferably in the range 10³ mPas to 10⁶ mPas.

10. The pharmaceutical composition as claimed in claim 9, **characterized in that** the increase in the viscosity occurs by salination in combination with a hydrolysis of the composition conditioned by the body fluid, in particular of the support, in particular of the support formed as a gel.

## Revendications

1. Composition pharmaceutique à usage topique pour le traitement de lésions parodontales ou péri-implantaires chez l'humain ou le mammifère, comprenant
- au moins un principe actif antiseptique,
- au moins un principe actif antibiotique, qui se trouve dans des capsules, qui après l'application libèrent le principe actif antibiotique de manière contrôlée dans le temps,
- au moins un excipient, dans lequel se trouve au moins une partie du principe actif antiseptique, à une température ambiante de 20°C, la composition faisant preuve d'une viscosité dynamique comprise entre 5 et 10⁵ mPas, de préférence entre 50 et 10⁴ mPas, de manière plus préférentielle, entre 100 et 10⁴ mPas.

2. Composition pharmaceutique selon la revendication 1, **caractérisée en ce que** l'au moins un principe actif antiseptique est
- un alcool et/ou
- un composé ammonium quaternaire et/ou
- un composé iodé et/ou
- un composé halogéné et/ou
- un composé contenant du mercure.

3. Composition pharmaceutique selon la revendication 1 ou 2, **caractérisée en ce que** l'au moins un principe actif antibiotique fait preuve de l'un des mécanismes d'action suivants :
- l'inhibition de la synthèse des parois cellulaires,
- l'inhibition de la biosynthèse des protéines sur le ribosome,
- l'action sur des acides nucléiques bactériens.

4. Composition pharmaceutique selon l'une quelconque des revendications 1 à 3, **caractérisée en ce que** les capsules sont des nanocapsules et/ou des microcapsules.

5. Composition pharmaceutique selon l'une quelconque des revendications 1 à 4, **caractérisée en ce que** les capsules sont constituées d'un polysaccharide.

6. Composition pharmaceutique selon l'une quelconque des revendications 1 à 5, **caractérisée en ce que** la libération de l'au moins un principe actif antibiotique contenu dans les capsules a lieu sur une période d'au moins 5 jours, de préférence, d'au moins 10 jours.

7. Composition pharmaceutique selon l'une quelconque des revendications 1 à 6, **caractérisée en ce que** pour une première part des capsules, une libération de l'au moins un principe actif antibiotique contenu a lieu sur une période comprise entre un jour et 10 jours à partir de l'application et/ou sur une deuxième part des capsules, une libération de l'au moins un principe actif antibiotique contenu a lieu sur une période comprise entre un jour et 20 jours et/ou sur une troisième part des capsules, une libération de l'au moins un principe actif antibiotique contenu a lieu sur une période comprise entre 10 jours et 20 jours.

8. Composition pharmaceutique selon l'une quelconque des revendications 1 à 7, **caractérisée en ce que** l'excipient est de préférence un gel ou une composition de corps gras et ou un mélange des substances précédemment citées.

9. Composition pharmaceutique selon l'une quelconque des revendications 1 à 8, **caractérisée en ce que** la viscosité de la composition augmente après l'application, suite à une interaction avec un fluide corporel et/ou la température corporelle, après l'intervention de l'interaction avec le fluide corporel et/ou la température corporelle, de préférence après une période de un jour à trois jours, la viscosité étant comprise entre 10³ mPas et 10⁶ mPas.

10. Composition pharmaceutique selon la revendication 9, **caractérisée en ce que** l'augmentation de la viscosité a lieu par une salinisation consécutive à une hydrolyse due au fluide corporel de la composition, notamment de l'excipient, notamment de l'excipient conçu sous la forme d'un gel.
